# EUROPÄISCHE PATENTSCHRIFT

(11) **EP 3 187 889 B1**
(45) Veröffentlichungstag und Bekanntmachung des Hinweises auf die Patenterteilung: **27.02.2019**
(21) Anmeldenummer: 17168190.1
(22) Anmeldetag: 26.04.2017
(51) Int. Cl.: G01R 33/34

(54) **ANPASSBARE MRI-LOKALSPULENANORDNUNG**
ADAPTABLE LOCAL MRI COIL ASSEMBLY
BOBINE LOCALE ADAPTABLE POUR IRM

(43) Veröffentlichungstag der Anmeldung: 05.07.2017
(73) Patentinhaber: Siemens Healthcare GmbH, 91052 Erlangen (DE)
(72) Erfinder: Zink, Stephan, 91054 Erlangen (DE)

(56) Entgegenhaltungen:
- DE-A1-102013 105 273
- DE-A1-102014 207 020
- US-B1- 6 975 115

## Beschreibung

Die Erfindung betrifft eine Lokalspulenanordnung und eine Magnetresonanzvorrichtung.

In der Medizintechnik stellen bildgebende Verfahren wichtige Hilfsmittel dar. So können mittels Magnetresonanz (MR) klinische Schnittbilder mit hohen und variablen Weichteilkontraste erstellt werden. Dabei werden typischerweise eine oder mehrere MR-Spulenvorrichtungen verwendet, die in der Regel eine oder mehrere Magnetresonanzantennen, oft auch Spulenelemente genannt, zum Senden und/oder Empfangen von hochfrequenten elektromagnetischen Signalen aufweisen.

Gerade für eine MR-Bildgebung peripherer Körperregionen wie Gelenke an Armen und Beinen werden oftmals spezielle MR-Spulenvorrichtungen, insbesondere Lokalspulenanordnungen, verwendet. Es ist wünschenswert, eine solche Lokalspulenanordnung optimal an die Größe und/oder Form der untersuchten Körperteile anpassen zu können. Durch einen dadurch erreichbaren guten Füllfaktor und/oder einen minimierten räumlicher Abstand zwischen Untersuchungsobjekt und Spulenelementen, die die Lokalspulenanordnung üblicherweise aufweist, lässt sich das Signal-Rausch-Verhältnis und somit die Bildqualität verbessern. Ist die Lokalspulenanordnung zu groß ausgebildet, nimmt das Bildrauschen zu. Ist sie dagegen zu klein ausgebildet, kann dies den Patientenkomfort beeinträchtigen, etwa indem sie einen unangenehmen Druck ausüben, oder sie ist gar nicht einsetzbar.

Bei den bekannten Lokalspulenanordnungen kann zwischen starren, auf die Körperregion angepasste Typen und flexiblen Typen unterschieden werden. Starre Lokalspulenanordnungen, die z.B. oftmals für Kopfuntersuchungen eingesetzt werden, sind möglichst optimal an eine durchschnittliche Patientenanatomie angepasst, um möglichst viele Patienten (üblich 95% Perzentile) damit abzudecken. Für Patienten mit kleinen oder mittleren Anatomieabmessungen wird jedoch beim Empfang nicht die optimal mögliche Bildqualität erreicht, da die Spulenelementen nicht optimal nahe am Körper des Patienten anliegen.

Flexible Lokalspulenanordnungen können diese fehlende Anpassung oftmals nur unter Inkaufnahme von Auswölbungen und Abstehen von Teilbereichen der Lokalspulenanordnung ausgleichen. Beispielsweise weisen bei zylindrischen Körperformen, wie z.B. bei Knie und Ellbogen, die Spulenelemente in der Regel einen unterschiedliche Überlapp oder eine unterschiedliche Lücke auf. Dies schränkt die erreichbare Bildqualität ein. Beispielsweise beschreibt die Offenlegungsschrift DE 10 2011 007 065 A1 eine Kniespule, die einen starren und einen flexiblen Teil umfasst. Die Offenlegungsschrift DE 10 2006 027 189 A1 zeigt eine Lokalspulenanordnung, die sowohl in der Höhe als auch in der Breite variiert werden kann. Die Offenlegungsschrift DE 10 2015 210 529 A1 offenbart eine Lokalspulenanordnung mit einer anpassbaren Trägerstruktur mit mehreren Trägergliedern, die eine geschlossene Kette bilden.

DE 10 2014 207 020 offenbart eine Lokalspulenanordnung zur Untersuchung eines Untersuchungsobjekts mittels Magnetresonanz umfassend drei Spuleneinheiten, wobei die Spuleneinheiten einen Objektaufnahmebereich zur Aufnahme des Untersuchungsobjekts zumindest teilweise umschließen, wobei durch Bewegungen zur Zentralachse der ausfahrbaren Spuleneinheiten die Form und/oder Größe des Objektaufnahmebereichs veränderbar ist.

Mit der vorliegenden Erfindung soll eine Magnetresonanzspulenvorrichtung bereitgestellt werden, die eine verbesserte Bildqualität einer Magnetresonanztomographie bei hohem Patientenkomfort ermöglicht.
Die Aufgabe wird durch die Merkmale der unabhängigen Ansprüche gelöst. Vorteilhafte Ausgestaltungen sind in den Unteransprüchen beschrieben.

Demnach wird eine Lokalspulenanordnung zur Untersuchung eines Untersuchungsobjekts, insbesondere eines im Wesentlichen zylinderförmigen Körperteils wie z.B. ein Knie, mittels Magnetresonanz vorgeschlagen, welche Lokalspulenanordnung zumindest eine erste und eine zweite Spuleneinheit sowie zumindest eine erste Zwischeneinheit umfasst. Die Lokalspulenanordnung kann also zusätzlich zur ersten und zweiten Spuleneinheit noch eine oder mehrere weitere Spuleneinheiten umfassen. Ebenso kann die Lokalspulenanordnung zusätzlich zur ersten Zwischeneinheit noch eine oder mehrere weitere Zwischeneinheiten, insbesondere eine zweite Zwischeneinheit, umfassen.

In einem montierten Zustand ist die erste Zwischeneinheit vorzugsweise zwischen der ersten Spuleneinheit und der zweiten Spuleneinheit angeordnet. Insbesondere sind Spuleneinheiten und Zwischeneinheiten durchgehend abwechselnd angeordnet, z.B. entlang eines Umfangs in folgender Reichenfolge: erste Spuleneinheit, erste Zwischeneinheit, zweite Spuleneinheit und zweite Zwischeneinheit, an die sich dann wieder die erste Spuleneinheit anschließt.

Dabei umschließt die erste Spuleneinheit, die zweite Spuleneinheit und die erste Zwischeneinheit einen Objektaufnahmebereich zur Aufnahme des Untersuchungsobjekts zumindest teilweise, wobei der Objektaufnahmebereich insbesondere eine Zentralachse aufweist. Der Objektaufnahmebereich ist vorzugsweise ausgebildet, darin ein Untersuchungsobjekt zum Zwecke einer MR-Untersuchung zu positionieren und/oder zu lagern. Der Objektaufnahmebereich kann dimensioniert sein, um beispielsweise ein Knie als Untersuchungsobjekt aufzunehmen. Insbesondere kann die Lokalspulenanordnung in einer Umfangsrichtung geschlossen ausgebildet sein, insbesondere falls die Lokalspulenanordnung ferner eine zweite Zwischeneinheit umfasst, und/oder in Richtung der Zentralachse an einer oder vorzugsweise zwei Seiten geöffnet sein, um beispielsweise ein Knie im Objektaufnahmebereich zu lagern.

Vorzugsweise sind durch Relativbewegungen senkrecht zur Zentralachse der ersten Spuleneinheit, der zweiten Spuleneinheit und der ersten Zwischeneinheit die Form und/oder Größe des Objektaufnahmebereichs veränderbar. Eine Richtung senkrecht zu einer Achse wird oftmals auch als eine radiale Richtung bezeichnet. Bei jeder dieser Relativbewegungen ist zumindest jeweils eine Komponente der Relativbewegung, insbesondere die komplette Relativbewegung, senkrecht zur Zentralachse, also radial, orientiert. Möglicherweise weist die Relativbewegung neben der Komponente senkrecht zur Zentralachse eine weitere Komponente parallel zu Zentralachse auf. Eine Richtung parallel zu einer Achse wird oftmals auch als eine axiale Richtung bezeichnet.

Die Bewegungen der ersten Spuleneinheit, der zweiten Spuleneinheit und der ersten Zwischeneinheit erfolgen also vorzugsweise relativ zur Zentralachse in senkrechter Richtung zur Zentralachse. Falls also die erste Spuleneinheit, die zweite Spuleneinheit und die erste Zwischeneinheit und eine etwaige zweite Zwischeneinheit senkrecht zur Zentralachse bewegt werden, ändert sich die Form und/oder Größe des Objektaufnahmebereichs. Dadurch kann die Lokalspulenanordnung an die individuelle Anatomie des Untersuchungsobjekts angepasst werden. Insbesondere kann eine Form und/oder Größe des Objektaufnahmebereichs in einer Querschnittsebene senkrecht zur Zentralachse angepasst werden. Beispielsweise kann die Lokalspulenanordnung sowohl für dicke als auch für dünne Knieformen verwendet werden.

Die erste Spuleneinheit, die zweite Spuleneinheit, die erste Zwischeneinheit und eine etwaige zweite Zwischeneinheit sind vorzugsweise relativ zueinander beweglich angeordnet. Sie vollziehen bei Relativbewegungen zur Zentralachse nicht nur Bewegungen relativ zur Zentralachse, sondern auch relativ zueinander. Insbesondere bewegen sich dabei die erste Spuleneinheit und die erste Zwischeneinheit relativ zueinander in einer ersten Verschiebungsrichtung. Insbesondere bewegen sich dabei die zweite Spuleneinheit und die erste Zwischeneinheit relativ zueinander in einer zweiten Verschiebungsrichtung.

Die Relativbewegungen der ersten Spuleneinheit, der zweiten Spuleneinheit und der ersten Zwischeneinheit sind zwangsgekoppelt, insbesondere zwangsgeführt. Eine Relativbewegung der ersten Spuleneinheit senkrecht zur Zentralachse geht also einher mit weiteren Relativbewegungen der zweiten Spuleneinheit und der ersten Zwischeneinheit senkrecht zur Zentralachse. Durch die Kopplung der Relativbewegungen relativ zur Zentralachse kann der Objektaufnahmebereich definiert und/oder reproduzierbar angepasst werden.

Vorzugsweise verändern sich bei Relativbewegungen senkrecht zur Zentralachse der ersten Spuleneinheit, der zweiten Spuleneinheit, der ersten Zwischeneinheit und einer etwaigen zweiten Zwischeneinheit die Abstände zwischen der Zentralachse und der ersten Spuleneinheit, der Zentralachse und der zweiten Spuleneinheit, der Zentralachse und der ersten Zwischeneinheit sowie der Zentralachse und der etwaigen zweiten Zwischeneinheit gleichsinnig, d.h. insbesondere bei einer Vergrößerung eines Abstands zwischen der ersten Spuleneinheit und der Zentralachse vergrößern sich auch Abstände zwischen der zweiten Spuleneinheit und der Zentralachse, zwischen der ersten Zwischeneinheit und der Zentralachse und zwischen der etwaigen zweiten Zwischeneinheit und der Zentralachse bzw. bei einer Verkleinerung eines Abstands zwischen der ersten Spuleneinheit und der Zentralachse verkleinern sich auch Abstände zwischen der zweiten Spuleneinheit und der Zentralachse, zwischen der ersten Zwischeneinheit und der Zentralachse und zwischen der etwaigen zweiten Zwischeneinheit und der Zentralachse. Gemäß einer besonders bevorzugten Ausführungsform verändern sich die Abstände der ersten und zweiten Spuleneinheit zur Zentralachse und/oder der ersten Zwischeneinheit zur Zentralachse durch deren Relativbewegungen nicht nur gleichsinnig, sondern jeweils um den gleichen Betrag.

Insbesondere falls die erste oder die zweite Spuleneinheit oder die erste Zwischeneinheit oder die etwaige zweite Zwischeneinheit fest in einem Raum angeordnet ist, d.h. bei unveränderlicher Position bezüglich eines Koordinatensystems und/oder Inertialsystems dieses Raums, bewegt sich bei einer Relativbewegung die Zentralachse in diesem Raum, so dass sich die Position der Zentralachse bezüglich dieses Koordinatensystems und/oder Inertialsystems verändert.

Vorzugsweise weisen die erste Spuleneinheit und die erste Zwischeneinheit jeweils eine Mittelebene auf, die sich in der

Zentralachse schneiden, wobei die Lokalspulenanordnung derart ausgebildet ist, dass die Relativbewegungen senkrecht zur Zentralachse der ersten Spuleneinheit und der ersten Zwischeneinheit parallel zur jeweiligen Mittelebene erfolgen, d.h. die Relativbewegung der ersten Spuleneinheit erfolgt also parallel zur Mittelebene der ersten Spuleneinheit und die Relativbewegung der ersten Zwischeneinheit erfolgt parallel zur Mittelebene der ersten Zwischeneinheit. Dadurch kann eine kontrollierte und gleichmäßige Anpassung der Form und/oder Größe des Objektaufnahmebereichs erfolgen.

Vorzugsweise ist zumindest eine der Mittelebenen eine Symmetrieebene, d.h. die erste Spuleneinheit und/oder die erste Zwischeneinheit ist symmetrisch ihrer Mittelebene ausgebildet. Auch etwaige weitere Spuleneinheiten und/oder Zwischeneinheiten können bezüglich einer jeweiligen Mittelebene symmetrisch ausgebildet sein. Dies ermöglicht einen besonders einfachen Aufbau der Lokalspulenanordnung.

Vorzugsweise sind die erste Spuleneinheit und/oder die zweite Spuleneinheit und/oder die erste Zwischeneinheit starr, insbesondere rigide, steif, biegesteif, unbiegsam und/oder unelastisch, ausgebildet. Auch etwaige weitere Spuleneinheiten und/oder Zwischeneinheiten können starr, insbesondere rigide, steif, biegesteif, unbiegsam und/oder unelastisch, ausgebildet sein. Die Änderung der Form und/oder Größe des Objektaufnahmebereichs erfolgt also nicht durch eine Änderung der Form der ersten Spuleneinheit und/oder der zweiten Spuleneinheit und/oder des erste Zwischeneinheit, sondern durch deren relative Bewegung zueinander. Dies ermöglicht eine höhere Kontrolle der Eigenschaften der Lokalspulenanordnung bei einer Form- und/oder Größenveränderung.

Vorzugsweise umfasst die Lokalspulenanordnung ein erstes Teil und ein zweites Teil, wobei das erste Teil die erste Spuleneinheit und die erste Zwischeneinheit umfasst, das zweite Teil die zweite Spuleneinheit umfasst und das erste Teil vom zweiten Teil demontierbar ist. Das erste Teil und/oder das zweite Teil können noch etwaige weitere Spuleneinheiten und/oder Zwischeneinheiten umfassen. Somit kann die Lokalspulenanordnung von einem montierten Zustand in einem demontierten Zustand überführt werden. Beispielsweise ist das erste Teil ein anteriores Teil der Lokalspulenanordnung und der zweite Teil ein posteriores Teil der Lokalspulenanordnung. Im demontierten Zustand kann das Untersuchungsobjekt besonders komfortabel positioniert werden, da beispielsweise ein Knie in einen posterioren Teil einer Kniespule hineingelegt werden kann, ohne dass dabei das anteriore Teil stört.

Die erste Spuleneinheit und/oder die zweite Spuleneinheit und/oder die erste Zwischeneinheit umfassen zumindest einen Teil einer Magnetresonanzantenne, insbesondere einer Empfangsantenne, welche oft auch RX-Antenne genannt wird. Beispielsweise weisen die erste Spuleneinheit einen ersten Teil einer ersten Magnetresonanzantenne auf und die zweite Spuleneinheit einen ersten Teil einer zweiten Magnetresonanzantenne auf, und die erste Zwischeneinheit weist einen zweiten Teil der ersten Magnetresonanzantenne und einen zweiten Teil der zweiten Magnetresonanzantenenne auf. Der erste Teil und der zweite Teil der ersten Magnetresonanzantenne und der erste und der zweite Teil der zweiten Magnetresonanzantenne wirken dabei vorzugsweise, insbesondere in einem montierten Zustand, jeweils als eine, insbesondere vollständige, Magnetresonanzantenne.

Sofern die Lokalspulenanordnung weitere Spuleneinheit und/oder Zwischeneinheiten umfasst, können diese selbstverständlich ebenso zumindest einen Teil einer Magnetresonanzantenne aufweisen. Die Spuleneinheiten und/oder Zwischeneinheit können auch mehrere Magnetresonanzantennen aufweisen.

Eine Magnetresonanzantenne umfasst üblicherweise eine elektrische Schleife, die möglicherweise mit einer elektrischen Schaltung verbunden ist, die beispielsweise ausgelegt ist, Magnetresonanzsignale zu empfangen und/oder Anregungssignale zu senden. Ein Teil einer Magnetresonanzantenne kann beispielsweise einen Teil einer elektrischen Schleife, z.B. eine Halbschleife, umfassen. Zwei Teile einer Magnetresonanzantenne können sich vorzugsweise zu einer, insbesondere vollständigen, Magnetresonanzantenne ergänzen.

Bevorzugt umfasst die erste Zwischeneinheit eine erste Kontakteinheit und eine zweite Kontakteinheit. Ferner umfassen die erste Spuleneinheit und die zweite Spuleneinheit jeweils zumindest eine weitere Kontakteinheit, wobei die erste Kontakteinheit der ersten Zwischeneinheit an der zumindest einen weiteren Kontakteinheit der ersten Spuleneinheit anordbar ist und die zweite Kontakteinheit der ersten Zwischeneinheit an der zumindest einen weiteren Kontakteinheit der zweiten Spuleneinheit anordbar ist. Dabei ist die erste Zwischeneinheit relativ zu der ersten Spuleneinheit und der zweiten Spuleneinheit entlang der aneinander anordbaren Kontakteinheiten bewegbar.

Vorzugsweise korrespondiert die zumindest eine weitere Kontakteinheit zur ersten oder zweiten Kontakteinheit, so dass diese aufeinander abgestimmt sind. Dazu können die Kontakteinheiten beispielsweise Punkte und/oder Flächen umfassen, die zumindest in einem montierten Zustand miteinander wechselwirken, insbesondere einander berühren.

Mit Hilfe der Kontakteinheiten kann die erste Zwischeneinheit mit der ersten und zweiten Spuleneinheit bewegbar verbunden werden. Über die Kontakteinheiten kann vorzugsweise zumindest ein elektrischer und/oder zumindest ein mechanischer Kontakt zwischen der erste Zwischeneinheit und der erste Spuleneinheit und/oder der zweiten Spuleneinheit hergestellt werden.

Somit können beispielsweise elektrische Signale übertragen werden. Durch den zumindest einen elektrischen Kontakt können beispielsweise Teile einer elektrischen Schleife miteinander verbunden werden, die insbesondere von einer Magnetresonanzantenne umfasst sein können.

Vorzugsweise weist die erste Zwischeneinheit einen ersten Teil einer Magnetresonanzantenne auf und die erste Spuleneinheit weist einen zweiten Teil der Magnetresonanzantenne auf, wobei der erste Teil und der zweite Teil der Magnetresonanzantenne durch die erste Kontakteinheit der ersten Zwischeneinheit und die zumindest eine weitere Kontakteinheit der ersten Spuleneinheit verbindbar sind. Damit kann erreicht werden, dass sich eine Magnetresonanzantenne über mehrere Einheiten der Lokalspulenanordnung erstreckt. Dadurch kann die messtechnische Abdeckung des Untersuchungsobjekts verbessert und das Signal-Rausch-Verhältnis gesteigert werden.

Durch den zumindest einen mechanischen Kontakt können beispielsweise mechanische Kräfte zwischen der erste Zwischeneinheit und der erste Spuleneinheit und/oder der zweiten Spuleneinheit übertragen werden, welche die Relativbewegungen senkrecht zur Zentralachse der ersten Spuleneinheit, der zweiten Spuleneinheit und der ersten Zwischeneinheit unterstützen, insbesondere führen.

Vorzugsweise weisen die erste Kontakteinheit der ersten Zwischeneinheit und die weitere Kontakteinheit der ersten Spuleneinheit eine aufeinander abgestimmt mechanische Führung ab, welche die Relativbewegungen zumindest teilweise führt. Vorzugsweise weisen die erste Kontakteinheit der ersten Zwischeneinheit und die weitere Kontakteinheit der zweiten Spuleneinheit eine aufeinander abgestimmt mechanische Führung ab, welche die Relativbewegungen zumindest teilweise führt. Eine solche mechanische Führung kann beispielsweise ein ineinandergreifendes Schienensystem umfassen.

Vorzugsweise weist die zumindest eine weitere Kontakteinheit der ersten Spuleneinheit und/oder der zweiten Spuleneinheit jeweils eine Oberfläche auf, die gegenüber einer Richtung der Relativbewegung zur Zentralachse, insbesondere der senkrechten Komponente der Relativbewegung zur Zentralachse, der ersten Zwischeneinheit um einen Neigungswinkel geneigt ist, wobei der Neigungswinkel größer als 0° und kleiner als 90° ist. Der Neigungswinkel ist insbesondere größer als 10° und kleiner als 80°. Der Neigungswinkel ist insbesondere größer als 20° und kleiner als 70°. Der Neigungswinkel ist insbesondere größer als 30° und kleiner als 60°. Der Neigungswinkel ist insbesondere größer als 40° und kleiner als 50°. Der Neigungswinkel ist insbesondere im Wesentlichen 45°.

Der Neigungswinkel korrespondiert vorzugsweise mit der Kopplung der Relativbewegungen relativ zur Zentralachse. Insbesondere korrespondiert ein Neigungswinkel von 45° mit Relativbewegungen senkrecht zur Zentralachse der ersten Spuleneinheit, der zweiten Spuleneinheit und der ersten Zwischeneinheit, bei der sich die Abstände zwischen der Zentralachse der ersten Spuleneinheit, der zweiten Spuleneinheit und der ersten Zwischeneinheit um einen gleichen Betrag ändern. Durch die Wahl des Neigungswinkels kann insbesondere eingestellt werden, wie sich die Form der Lokalspulenanordnung in einer Querschnittsebene senkrecht zur Zentralachse bei einer Relativbewegung der ersten Spuleneinheit, der zweiten Spuleneinheit und der ersten Zwischeneinheit ändert.

Vorzugsweise befindet sich die besagte jeweils eine Oberfläche der zumindest einen weiteren Kontakteinheit der ersten Spuleneinheit und/oder der zweiten Spuleneinheit auf der der Zentralachse zugewandten Seite der ersten Spuleneinheit und/oder der zweiten Spuleneinheit. Vorzugsweise begrenzt die besagte jeweils eine Oberfläche der zumindest einen weiteren Kontakteinheit der ersten Spuleneinheit und/oder der zweiten Spuleneinheit den Objektaufnahmebereich.

Vorzugsweise weist die erste Kontakteinheit und/oder die zweite Kontakteinheit der ersten Zwischeneinheit eine Oberfläche auf, die in einem montierten Zustand der Lokalspulenanordnung der besagten Oberfläche der zumindest einen weiteren Kontakteinheit der ersten Spuleneinheit und/oder der zweiten Spuleneinheit gegenüberliegt, insbesondere parallel dazu verläuft.

Vorzugsweise umfasst erste Kontakteinheit und/oder die zweite Kontakteinheit und/oder die zumindest eine weitere Kontakteinheit zumindest eine Kontaktfläche. Vorzugsweise ist die zumindest eine Kontaktfläche auf der besagten Oberfläche der der zumindest einen weiteren Kontakteinheit der ersten Spuleneinheit und/oder der zweiten Spuleneinheit angeordnet.

Vorzugsweise umfasst die erste Kontakteinheit und die zweite Kontakteinheit der ersten Zwischeneinheit sowie die jeweils zumindest eine weitere Kontakteinheit der ersten Spuleneinheit und der zweiten Spuleneinheit jeweils ein elektrisches Verbindungselement. Damit kann insbesondere der bereits erwähnte elektrische Kontakt zwischen der erste Zwischeneinheit und der erste Spuleneinheit und/oder der zweiten Spuleneinheit hergestellt werden, um beispielsweise Teil einer Magnetresonanzantenne miteinander zu verbinden.

Vorzugsweise umfasst das elektrische Verbindungselement der ersten Kontakteinheit und/oder der zweiten Kontakteinheit der ersten Zwischeneinheit und/oder der zumindest einen weiteren Kontakteinheit der ersten Spuleneinheit und/oder der zweiten Spuleneinheit zumindest eine Landefläche zur Aufnahme eines Gegenkontakts. Unter einer Landefläche kann insbesondere eine elektrisch leitfähige Fläche verstanden werden, auf der ein elektrisch leitfähiger Gegenkontakt, z.B. ein Pin, positioniert werden kann, um insbesondere einen Stromkreis zu schließen.

Vorzugsweise umfasst der Gegenkontakt zumindest einen gefederten Kontakt und/oder Kugelkontakt und/oder Schleifkontakt. Mit diesen Kontaktvarianten können besonders zuverlässige elektrische Verbindungen hergestellt werden. Insbesondere kann ein gefederter Kontakt Fertigungstoleranzen ausgleichen.

Vorzugsweise umfasst das elektrische Verbindungselement der ersten Kontakteinheit und/oder der zweiten Kontakteinheit der ersten Zwischeneinheit und/oder der zumindest einen weiteren Kontakteinheit der ersten Spuleneinheit und/oder der zweiten Spuleneinheit mehrere Landeflächen zur Aufnahme eines Gegenkontakts, wobei die mehreren Landeflächen linear entlang der ersten oder zweiten Verschiebungsrichtung angeordnet sind. Durch eine solche Anordnung der mehreren Landeflächen kann insbesondere die Größe der elektrischen Schleifen verändert werden bei gleichzeitiger Sicherstellung eines elektrischen Kontakts.

Vorzugsweise umfasst das elektrische Verbindungselement der ersten Kontakteinheit und/oder der zweiten Kontakteinheit der ersten Zwischeneinheit und/oder der zumindest einen weiteren Kontakteinheit der ersten Spuleneinheit und/oder der zweiten Spuleneinheit zumindest eine Korrekturschaltung, wobei das elektrische Verbindungselement ausgebildet ist, abhängig von einer Kontaktierung der mehreren Landeflächen durch den Gegenkontakt die zumindest eine Korrekturschaltung zu aktivieren. Je nachdem, welche der mehreren Landeflächen durch den Gegenkontakt kontaktiert wird, wirkt vorzugsweise eine, insbesondere auf Größe der daraus resultierenden Schleife, angepasste Korrekturschaltung. Damit kann die Qualität etwaiger durch die Schleife empfangener Magnetresonanzsignale erhöht werden.

Eine Ausführungsform der Lokalspulenanordnung sieht vor, dass die Lokalspulenanordnung eine zweite Zwischeneinheit umfasst, wobei in einem montierten Zustand die zweite Zwischeneinheit zwischen der ersten Spuleneinheit und der zweiten Spuleneinheit angeordnet ist, wobei die erste Zwischeneinheit und die zweite Zwischeneinheit auf einander gegenüberliegenden Seiten der Lokalspulenanordnung angeordnet sind. Diese Anordnung stellt einen besonders einfachen Aufbau der Lokalspulenanordnung dar. Damit kann die Lokalspulenanordnung sowohl durch Veränderung des Abstandes zwischen der ersten Spuleneinheit und der zweiten Spuleneinheit in einer Höhe als auch durch Veränderung des Abstandes zwischen der ersten Zwischeneinheit und der zweiten Zwischeneinheit in einer Breite verändert werden.

Die Lokalspulenanordnung weist vorzugsweise mehrere Zwischeneinheiten und mehrere Spuleneinheiten auf, wobei die Anzahl der mehreren Zwischeneinheiten gleich der Anzahl der mehreren Spuleneinheiten ist. Vorzugsweise ist in einem montierten Zustand jede Zwischeneinheit zwischen zwei Spuleneinheiten angeordnet.

Vorzugsweise umfasst die Lokalspulenanordnung ein Gehäuse mit einer Außenoberfläche, das derart ausgebildet ist, dass es seine Form und Größe bei Relativbewegungen senkrecht zur Zentralachse der ersten Spuleneinheit, der zweiten Spuleneinheit und der ersten Zwischeneinheit unverändert bleibt. Vorzugsweise ist die Außenoberfläche auf einer Seite der Lokalspulenanordnung angeordnet, die der Zentralachse abgewandt ist.

Vorzugsweise umfasst das Gehäuse zumindest eine Magnetresonanzantenne zum Senden von HF-Signalen. Unter HF-Signalen sind vorzugsweise hochfrequente elektromagnetische Signale zur Anregung von Atomkernen im Untersuchungsobjekt zu verstehen.

Ferner wird eine Magnetresonanzvorrichtung vorgeschlagen mit zumindest einer erfindungsgemäßen Lokalspulenanordnung.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine Magnetresonanzvorrichtung mit einer Lokalspulenanordnung in einer schematischen Darstellung,
- Fig. 2: eine Lokalspulenanordnung mit einem größeren Objektaufnahmebereich,
- Fig. 3: eine Lokalspulenanordnung mit einem kleineren Objektaufnahmebereich,
- Fig. 4: eine Lokalspulenanordnung in einer perspektivischen Darstellung,
- Fig. 5: eine Lokalspulenanordnung in einer Querschnittsdarstellung,
- Fig. 6: ein elektrisches Verbindungselement mit in Reihe geschalteten Korrekturschaltungen,
- Fig. 7: ein elektrisches Verbindungselement mit parallel geschalteten Korrekturschaltungen,
- Fig. 8: eine Lokalspulenanordnung mit jeweils drei Zwischeneinheiten und Spuleneinheiten in einer schematischen Darstellung.

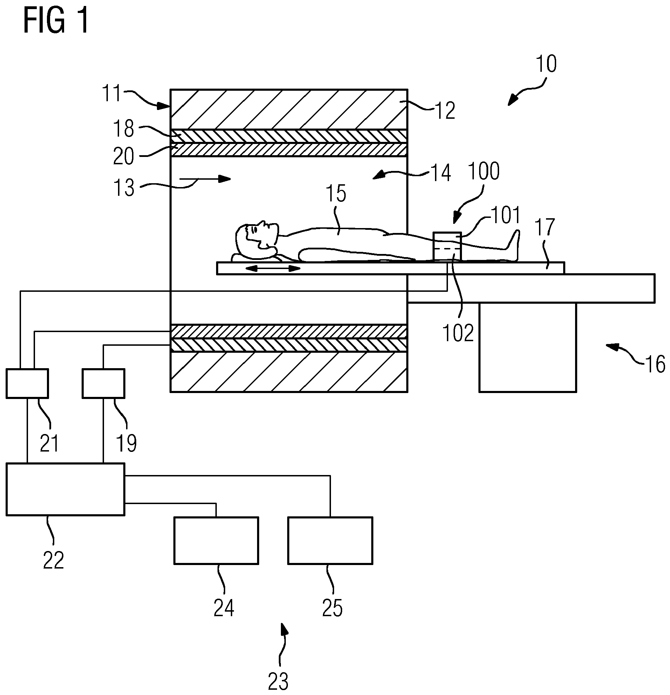

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum ein, der im Wesentlichen von einem Patientenaufnahmebereich 14 der Magnetresonanzvorrichtung 10 gebildet ist. Die Hochfrequenzantenneneinheit 20 ist weiterhin zum Empfang von Magnetresonanzsignalen ausgebildet.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

In diesem Beispiel soll ein Knie des Patienten 15 als Untersuchungsobjekt untersucht werden, weshalb am Knie eine Lokalspulenanordnung 100 zur Untersuchung des Untersuchungsobjekts mittels Magnetresonanz angeordnet ist. Die Lokalspulenanordnung 100 ist insbesondere ausgebildet, Magnetresonanzsignale zu empfangen und an die Hochfrequenzantennensteuereinheit 21 zur weiteren Verarbeitung weiterzuleiten.

Die Lokalspulenanordnung 100 umfasst hier ein erstes (anteriores) Teil 101 und ein zweites (posteriores) Teil 102, wobei das erste Teil 101 von dem zweiten Teil 102 trennbar ausgeführt ist, so dass das Knie bequem in einem demontierten Zustand auf das zweite Teil 102 gelegt werden kann und in einem weiteren Schritt das erste Teil 101 auf zweite Teil montiert werden kann, um den in Fig. 1 dargestellten montierten Zustand der Lokalspulenanordnung herbeizuführen. Mit anderen Worten kann ein durchgängige Lokalspulenanordnung geöffnet werden, um ein Körperteil zu platzieren, und dann wieder verschlossen werden.

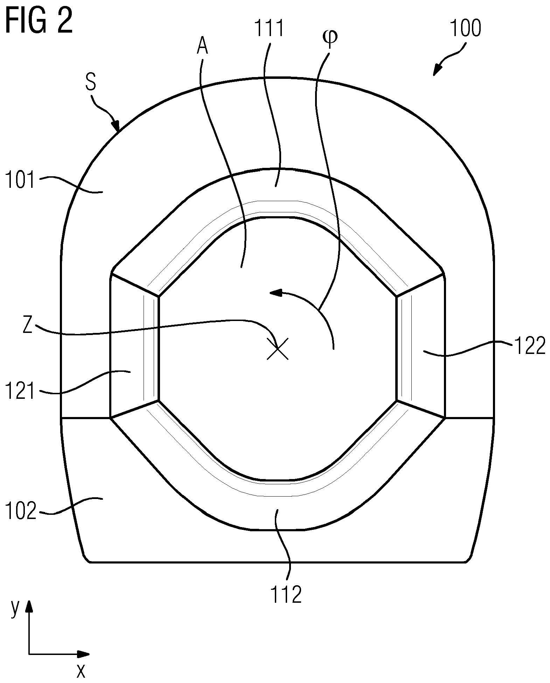

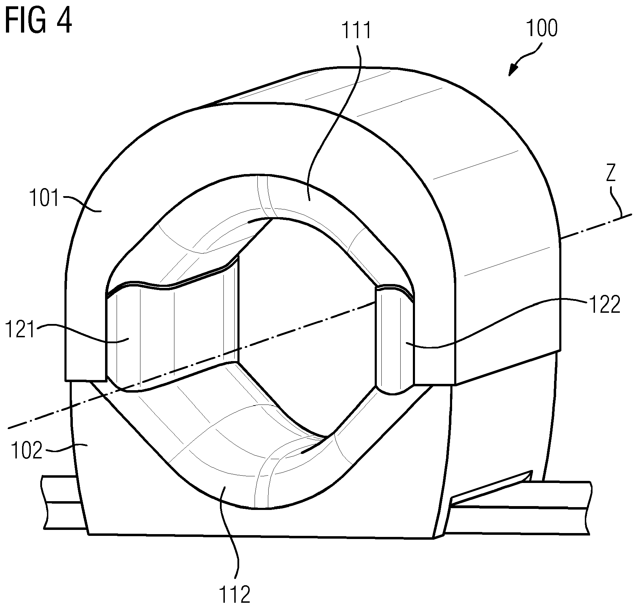

In den Fig. 2, 3 und 4 ist die Lokalspulenanordnung 100 beispielhaft in einem montierten Zustand dargestellt, wobei Fig. 4 eine perspektivische Darstellung zeigt. Die Lokalspulenanordnung 100 umfasst ein ersten Spuleneinheit 111 und eine zweite Spuleneinheit 112, eine erste Zwischeneinheit 121 und eine zweite Zwischeneinheit 122. Dabei wird die erste Spuleneinheit 111 und die erste Zwischeneinheit 121 (ebenso wie die zweite Zwischeneinheit 122) vom ersten Teil 101 und die zweite Spuleneinheit 112 vom zweiten Teil 102 der Lokalspulenanordnung 100 umfasst.

In diesem Beispiel sind einem montierten Zustand die erste Zwischeneinheit 121 und die zweite Zwischeneinheit 122 jeweils zwischen der ersten Spuleneinheit 111 und der zweiten Spuleneinheit 112 angeordnet, jedoch auf einander gegenüberliegenden Seiten der Lokalspulenanordnung.

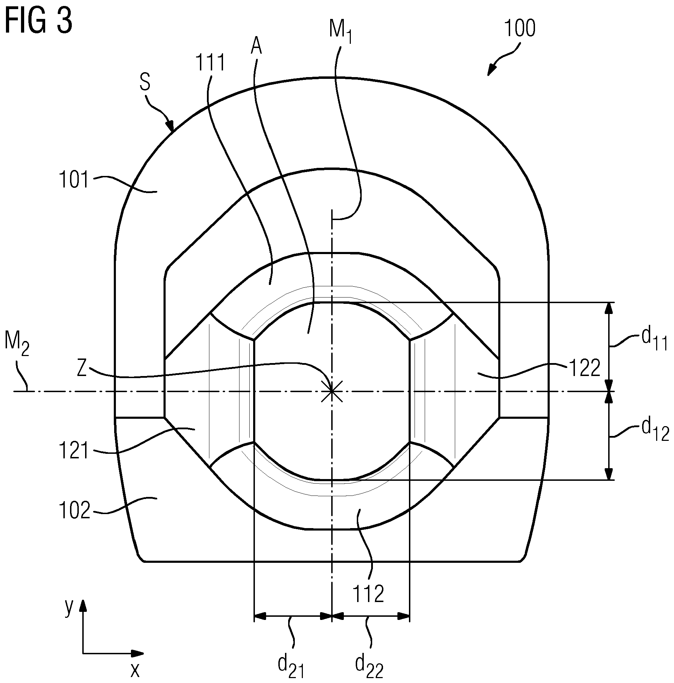

Die erste Spuleneinheit 111, die zweite Spuleneinheit 112 und die erste Zwischeneinheit 121 umschließen einen Objektaufnahmebereich A zur Aufnahme des Untersuchungsobjekts zumindest teilweise. Zusammen mit der zweiten Zwischeneinheit 122 wird der Objektaufnahmebereich A in Umfangsrichtung ϕ vollständig umschlossen. Der Objektaufnahmebereich A weist eine Zentralachse Z auf, die in der Darstellung von Fig. 2 und 3 senkrecht zur Zeichenebene orientiert ist.

Ein Vergleich der Fig. 2 mit Fig. 3 zeigt, dass durch Relativbewegungen senkrecht zur Zentralachse Z der ersten Spuleneinheit 111, der zweiten Spuleneinheit 112 und der ersten Zwischeneinheit 121 die Form und/oder Größe des Objektaufnahmebereichs A veränderbar ist. Die Veränderung des Objektaufnahmebereichs A erfolgt hier durch eine relative Bewegung der ersten Spuleneinheit 111 in einer senkrechten Richtung zur Zentralachse, durch eine weitere relative Bewegung der zweiten Spuleneinheit 112 in einer senkrechten Richtung zur Zentralachse und durch eine weitere relative Bewegung der ersten Zwischeneinheit 121 in einer senkrechten Richtung zur Zentralachse. Ferner bewegt sich in diesem Ausführungsbeispiel auch die zweite Zwischeneinheit 122 in einer senkrechten Richtung zur Zentralachse.

In Relation zu einem xy-Koordinatensystem, das ortsfest zum zweiten Teil 102 ist, verändern sich durch die Relativbewegungen der ersten Spuleneinheit 111, der zweiten Spuleneinheit 112 und der ersten Zwischeneinheit 121 die Koordinaten der Zentralachse Z. Bezüglich des xy-Koordinatensystems bleibt die zweite Spuleneinheit 112 statisch, während sich die erste Spuleneinheit 111 (nach oben oder unten) entlang der y-Achse bewegt. Bei dieser Bewegung nach unten nimmt die erste Spuleneinheit 111 die erste Zwischeneinheit 121 und die zweite Zwischeneinheit 122 mit, so dass sich die oben beschriebenen Relativbewegungen der ersten Spuleneinheit 111, der ersten Zwischeneinheit 121 und der zweiten Zwischeneinheit 122 relativ zur Zentralachse Z ergeben.

Ferner ändern sich bei Relativbewegungen senkrecht zur Zentralachse Z der ersten Spuleneinheit 111, der zweiten Spuleneinheit 112, der ersten Zwischeneinheit 121 und der zweiten Zwischeneinheit 122 die Abstände d₁₁, d₁₂, d₂₁, d₂₂ zwischen der Zentralachse Z und der ersten Spuleneinheit 111, der Zentralachse Z und der zweiten Spuleneinheit 112, der Zentralachse Z der ersten Zwischeneinheit 121 sowie der der Zentralachse Z der zweiten Zwischeneinheit 122 gleichsinnig, d.h. alle Abstände d₁₁, d₁₂, d₂₁ werden entweder größer oder alle Abstände d₁₁, d₁₂, d₂₁ werden kleiner. Somit können Untersuchungsobjekte mit verschiedenen Durchmessern möglichst enganliegend umschlossen werden. Insbesondere können bei Wiederholungsmessungen des gleichen Patienten 15 eine gleiche Größe und/oder Form des Objektaufnahmebereichs A eingestellt werden, so dass die damit durchführbare Magnetresonanzmessung besonders reproduzierbar ist.

Die erste Spuleneinheit 111 weist eine Mittelebene M₁ auf und die erste Zwischeneinheit weist eine Mittelebene M₂ auf. Die Mittelebenen M₁ und M₂ schneiden sich in der Zentralachse Z. Die Relativbewegungen senkrecht zur Zentralachse der ersten Spuleneinheit 111 und der ersten Zwischeneinheit 121, die insbesondere zur Veränderung der Form und/oder Größe des Objektaufnahmebereichs führen, erfolgen parallel zur jeweiligen Mittelebene, d.h. die Relativbewegung der ersten Spuleneinheit 111 erfolgt parallel zur Mittelebene M₁ und die Relativbewegung der ersten Zwischeneinheit 121 erfolgt parallel zur Mittelebene M₂.

Durch diesen Verstellmechanismus des Objektaufnahmebereichs A kommt wandert die Begrenzung des Objektaufnahmebereichs A in Bereiche, in denen noch Freiraum ist. Quetschungen, Klemmen, aber auch Verschmutzung kann dadurch vermieden werden. Zudem wird vorteilhafterweise der Arbeitsablauf vereinfacht, weil neben der Anpassung des Objektaufnahmebereichs A auch gleichzeitig durch die den Objektaufnahmebereich A begrenzende Innenfläche der Lokalspulenanordnung 100 eine Fixierung des Untersuchungsobjekts erfolgen kann. Mit der mehr oder weniger flexiblen Größe wird zudem funktionelle Bildgebung ermöglicht. Ferner kann die Lokalspulenanordnung 100 besonders flexibel angewendet werden, da diese auf unterschiedliche Geometrien des Untersuchungsobjekts (z.B. große und kleine Knie, Verbände, besondere Winkel von Gelenken, usw.) angepasst werden kann.

Die Lokalspulenanordnung 100 umfasst ein Gehäuse mit einer Außenoberfläche S, das derart ausgebildet, dass es seine Form und Größe bei Relativbewegungen senkrecht zur Zentralachse Z der ersten Spuleneinheit 111, der zweiten Spuleneinheit 112 und der ersten Zwischeneinheit 121 unverändert bleibt. Die Form der Umhausung der Lokalspulenanordnung 100 bleibt somit unabhängig von der Einstellung des Objektaufnahmebereichs A immer gleich, so dass sich dabei nur das Innenleben verstellt. Somit können beispielsweise leicht Magnetresonanzantennen zum Senden von HF-Signalen, oft auch TX-Antennen genannt, implementiert werden, so dass sich diese deren Position relativ zum xy-Koordinatensystem nicht verändert. Es ist aber auch denkbar, dass sie derart implementiert werden, dass sie sich bei einer Veränderung des Objektaufnahmebereichs A nachgeführt werden.

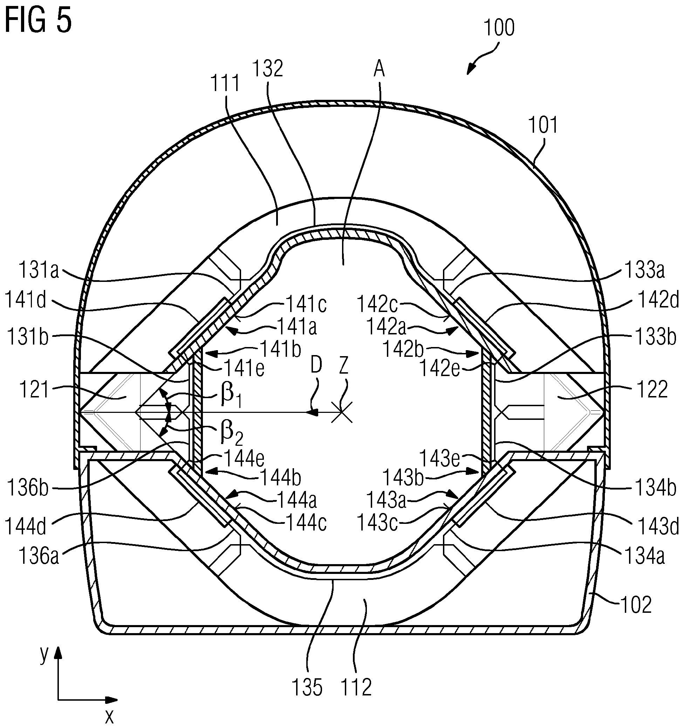

Fig. 5 zeigt eine beispielhafte Lokalspulenanordnung 100 in einem Querschnitt. Die erste Zwischeneinheit 121 umfasst eine erste Kontakteinheit 141b und eine zweite Kontakteinheit 144b. Die zweite Zwischeneinheit 122 umfasst eine Kontakteinheit 142b und eine Kontakteinheit 143b. Die erste Spuleneinheit 111 umfasst weitere Kontakteinheiten 141a und 142a. Die zweite Spuleneinheit 112 umfasst weitere Kontakteinheiten 143a und 144a. Die erste Kontakteinheit 141b der ersten Zwischeneinheit 121 ist an der Kontakteinheit 141a der ersten Spuleneinheit 111 angeordnet. Die zweite Kontakteinheit 144b der ersten Zwischeneinheit 121 ist an der Kontakteinheit 144a der zweiten Spuleneinheit 112 angeordnet. Dabei ist die Zwischeneinheit 121 relativ zu der ersten Spuleneinheit 111 und der zweiten Spuleneinheit 112 entlang der aneinander angeordneten Kontakteinheiten bewegbar, wie auch schon bereits anhand der Fig. 2 und 3 gezeigt wurde.

Die Kontakteinheit 141a der ersten Spuleneinheit 111 weist eine Oberfläche 141c auf und die Kontakteinheit 144a der zweiten Spuleneinheit 112 weist eine Oberfläche 144c auf. Die Oberfläche 141c ist gegenüber einer Richtung D der Relativbewegung zur Zentralachse Z der ersten Zwischeneinheit 121 um einen Neigungswinkel β₁ geneigt. Der Neigungswinkel β₁ ist größer als 0° und kleiner als 90°. Die Oberfläche 144c ist gegenüber der Richtung D der Relativbewegung zur Zentralachse Z der ersten Zwischeneinheit 121 um einen Neigungswinkel β₂ geneigt. Der Neigungswinkel β₂ ist größer als 0° und kleiner als 90°. Vorzugsweise ist β₁ gleich β₂.

Über den Neigungswinkel sind gezielt Anpassungsrichtungen bevorzugbar und/oder skalierbar. So bei einem Winkel von 45° ändert sich beispielsweise die Ausdehnung des Objektaufnahmebereichs A in x-Richtung (x-Ausdehnung, Breite) um den gleichen Betrag wie die Ausdehnung des Objektaufnahmebereichs A in y-Richtung (y-Ausdehnung, Höhe). Je größer der Neigungswinkel gewählt wird, desto stärker verändert sich die y-Ausdehnung im Vergleich zur x-Ausdehnung.

Auf der Oberfläche 141c kann die Kontakteinheit 141b bewegt werden und auf der 144c kann die Kontakteinheit 144b bewegt werden. Mit anderen Worten wandert auf der schrägen Oberfläche 141c die Kontakteinheit 141b auf und ab. Analoges gilt natürlich auch für die Oberflächen 142c und 143c mit ihren jeweiligen korrespondierenden Kontakteinheiten 142b und 143b. Daraus ergibt sich, dass die Relativbewegungen senkrecht zur Zentralachse Z der ersten Spuleneinheit 111, der zweiten Spuleneinheit 112 und der ersten Zwischeneinheit 121 gekoppelt sind.

Ein spiegelbildlicher Mechanismus ist bezüglich der zweiten Zwischeneinheit 121 und der ersten Spuleneinheit 111 bzw. der zweiten Spuleneinheit 112 gegeben.

Aus Fig. 5 ist ferner ersichtlich, dass in diesem Ausführungsbeispiel die erste Spuleneinheit 111, die zweite Spuleneinheit 112 und die erste Zwischeneinheit 121 (ebenso wie die zweite Zwischeneinheit 122) zumindest einen Teil einer Magnetresonanzantenne aufweist.

Die erste Zwischeneinheit 121 weist einen ersten Teil 131b einer ersten Magnetresonanzantenne 131 und einen ersten Teil 136b einer sechsten Magnetresonanzantenne 136 auf. Die zweite Zwischeneinheit 122 weist einen ersten Teil 133b einer dritten Magnetresonanzantenne 133 und einen ersten Teil 134b einer vierten Magnetresonanzantenne 134 auf.

Die erste Spuleneinheit 111 weist einen zweiten Teil 131a der ersten Magnetresonanzantenne 131, eine zweite Magnetresonanzantenne 132 und einen zweiten Teil 133a der dritten Magnetresonanzantenne 133 auf. Die zweite Spuleneinheit 112 weist einen zweiten Teil 134a der vierten Magnetresonanzantenne 134, eine fünfte Magnetresonanzantenne 135 und einen zweiten Teil 136a der sechsten Magnetresonanzantenne 136 auf.

Der erste Teil 131b und der zweite Teil 131a der ersten Magnetresonanzantenne 131 ist durch die erste Kontakteinheit 141b der ersten Zwischeneinheit 121 und die Kontakteinheit 141a der ersten Spuleneinheit 111 verbindbar. Ferner ist der erste Teil 133b und der zweite Teil 133a der dritten Magnetresonanzantenne 133 durch die Kontakteinheiten 142b und 142a verbindbar. Ferner ist der erste Teil 134b und der zweite Teil 134a der vierten Magnetresonanzantenne 134 durch die Kontakteinheiten 143b und 143a verbindbar. Ferner ist der erste Teil 136b und der zweite Teil 136a der sechsten Magnetresonanzantenne 136 durch die Kontakteinheiten 144b und 144a verbindbar.

Natürlich kann eine erfindungsgemäße Lokalspulenanordnung auch mehr oder weniger als sechs Magnetresonanzantennen umfassen, wie sie hier beispielhaft dargestellt sind.

Durch den hier beschriebenen Verstellmechanismus der des Objektaufnahmebereichs A können die geteilten Magnetresonanzantennen 131, 133, 134, 136 reproduzierbar in ihrer Form und/oder Größe verändert werden. Ein undefinierter Überlapp oder eine Lücke können vermieden werden.

Die erste Kontakteinheit 141b der ersten Zwischeneinheit 121 umfasst ein elektrisches Verbindungselement 141e. Die zweite Kontakteinheit 144b der ersten Zwischeneinheit 121 umfasst ein elektrisches Verbindungselement 144e. Ebenso umfassen die weiteren Kontakteinheiten 141a, 142a, 143a, 144a der ersten Spuleneinheit 111 und der zweiten Spuleneinheit 121 jeweils ein elektrisches Verbindungselement 141d, 142d, 143d, 144d. Auch die Kontakteinheiten 142b, 143b der zweiten Zwischeneinheit 122 umfassen jeweils ein elektrisches Verbindungselement 142e, 143e.

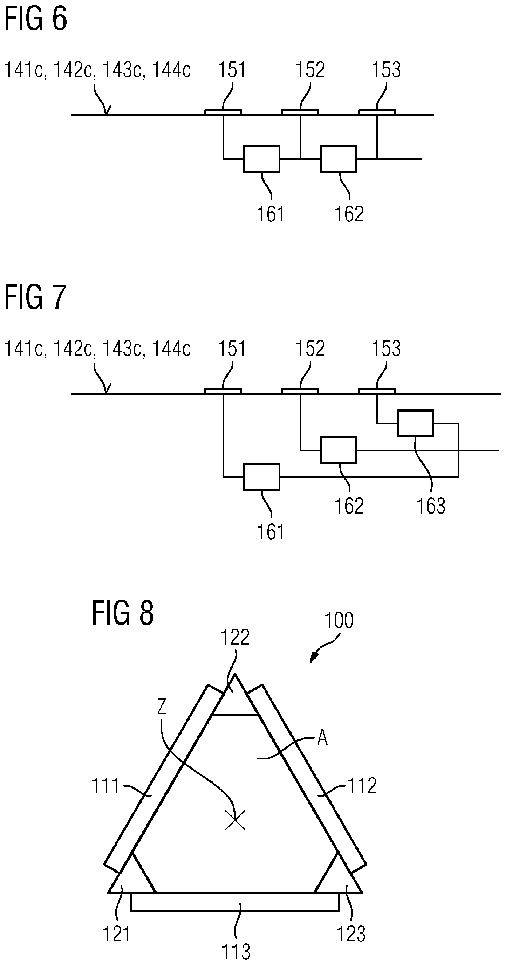

Fig. 6 und 7 zeigen eine schematische Darstellungen der elektrischen Verbindungselemente 141d, 142d, 143d, 144d, welche hier drei Landeflächen 151, 152, 153 zur Aufnahme eines Gegenkontakts umfassen. Die Landeflächen 151, 152, 153 sind in diesem Beispiel auf den Oberflächen 141c, 142c, 142c, 142d der Kontakteinheiten 141a, 142a, 143a, 144a angeordnet. In Fig. 6 sind die Landeflächen 151, 152, 153 mit Korrekturschaltungen 161, 162 verbunden, welche in Reihe geschalten sind. Eine andere Variante ist in Fig. 7 dargestellt, in der die Landeflächen 151, 152, 153 mit Korrekturschaltungen 161, 162, 163 verbunden, welche parallel geschalten sind.

Vorteilhafterweise sind elektrische Verbindungselemente 141d, 142d, 143d, 144d ausgebildet, abhängig von einer Kontaktierung der mehreren Landeflächen durch den Gegenkontakt eine der Korrekturschaltungen 161, 162, 163 zu aktivieren. Falls also der Gegenkontakt die Landefläche 151 kontaktiert, wird die Korrekturschaltungen 161 aktiviert, falls der Gegenkontakt die Landefläche 152 kontaktiert, wird die Korrekturschaltungen 163 aktiviert usw.

In diesem Ausführungsbeispiel umfassen die elektrischen Verbindungselemente 141e, 144e, 142e, 143e der ersten Zwischeneinheit 121 bzw. der zweiten Zwischeneinheit 122 jeweils einen Gegenkontakt, der hier als gefederter Schleifkontakt ausgebildet ist.

Bei einer Bewegung beispielsweise der ersten Zwischeneinheit 121 relativ zu der ersten Spuleneinheit 111 bewegt sich das elektrische Verbindungselement 141e, insbesondere der gefederte Schleifkontakt, entlang der Oberfläche 141c über die elektrische Verbindungseinheit 141d, insbesondere über die Landeflächen 151, 152, 153. Je nachdem, welche Form und/oder Größe des Objektaufnahmebereichs eingestellt wird, variiert auch die Größe der Magnetresonanzantenne 131. Die hiermit einhergehende Veränderung der Abstimmung der Magnetresonanzantenne 131 wird über eine Korrekturschaltung kompensiert, indem das Verbindungselement 141e eben diejenige der Landefläche 151, 152 oder 153 kontaktiert, die die dazu geeignete Korrekturschaltung 161, 162 oder 163 aktiviert.

Die größenveränderlichen Magnetresonanzantennen 131, 133, 134, 136 der Lokalspulenanordnung 100 können somit ohne oder mit nur minimalen Verlusten eine Körperregion als Untersuchungsobjekt umschließen, weil die Magnetresonanzantennen 131, 133, 134, 136 für jede Form und/oder Größe des Objektaufnahmebereichs A immer optimal abgestimmt sind.

Fig. 8 soll exemplarisch zeigen, dass der in Fig. 2 bis 5 dargestellte Verstellmechanismus auch auf eine größere Anzahl an Spuleneinheiten und Zwischeneinheiten übertragen werden kann. Hier umfasst die Lokalspulenanordnung 100 drei Spuleneinheiten 111, 112, 113 sowie drei Zwischeneinheiten 121, 122, 123, die jeweils zwischen den Spuleneinheiten 111, 112, 113 angeordnet sind. Sie umschließen zusammen einen Objektaufnahmebereich A mit einer Zentralachse Z.

Es wird abschließend noch einmal darauf hingewiesen, dass es sich bei den vorhergehend detailliert beschriebenen Verfahren sowie bei der dargestellten Erfassungsmustererzeugungseinheit und Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Lokalspulenanordnung (100) zur Untersuchung eines Untersuchungsobjekts mittels Magnetresonanz umfassend
- zumindest eine erste (111) und eine zweite Spuleneinheit (112),
- zumindest eine starre erste Zwischeneinheit (121),
wobei in einem montierten Zustand die erste Zwischeneinheit zwischen der ersten Spuleneinheit (111) und der zweiten Spuleneinheit (112) angeordnet ist,
wobei die erste Spuleneinheit (111), die zweite Spuleneinheit (112) und die erste Zwischeneinheit einen Objektaufnahmebereich (A) zur Aufnahme des Untersuchungsobjekts zumindest teilweise umschließen,
wobei der Objektaufnahmebereich (A) eine Zentralachse (Z) aufweist,
wobei die erste Spuleneinheit (111) und/oder die zweite Spuleneinheit (112) und/oder die erste Zwischeneinheit (121) zumindest einen Teil einer Magnetresonanzantenne (131-136) aufweist,
**dadurch gekennzeichnet,**
**dass** wobei die relativen Bewegungen der ersten Spuleneinheit (111), der zweiten Spuleneinheit (112) und der ersten Zwischeneinheit (121) derart zwangsgekoppelt sind,
**dass** die Form und/oder Größe des Objektaufnahmebereichs (A) veränderbar ist durch eine relative Bewegung der ersten Spuleneinheit (111) in einer senkrechten Richtung zur Zentralachse (Z), durch eine weitere relative Bewegung der zweiten Spuleneinheit (112) in einer senkrechten Richtung zur Zentralachse (Z) und durch eine weitere relative Bewegung der ersten Zwischeneinheit (121) in einer senkrechten Richtung zur Zentralachse (Z),
wobei sich die relativen Bewegungen der ersten Spuleneinheit (111), der zweiten Spuleneinheit (112) und der ersten Zwischeneinheit (121) relativ zueinander vollziehen.

2. Lokalspulenanordnung (100) nach einem der vorangehenden Ansprüche,
wobei sich bei Relativbewegungen senkrecht zur Zentralachse (Z) der ersten Spuleneinheit (111), der zweiten Spuleneinheit (112) und der ersten Zwischeneinheit (121) die Abstände zwischen
- der Zentralachse (Z) und der ersten Spuleneinheit (111),
- der Zentralachse (Z) und der zweiten Spuleneinheit (112) sowie
- der Zentralachse (Z) der ersten Zwischeneinheit (121) gleichsinnig ändern.

3. Lokalspulenanordnung (100) nach einem der vorangehenden Ansprüche,
wobei die erste Spuleneinheit (111) und die erste Zwischeneinheit (121) jeweils eine Mittelebene (M₁, M₂) aufweisen, die sich in der Zentralachse (Z) schneiden,
wobei die Relativbewegungen senkrecht zur Zentralachse (Z) der ersten Spuleneinheit (111) und der ersten Zwischeneinheit (121) parallel zur jeweiligen Mittelebene (M₁, M₂) erfolgen.

4. Lokalspulenanordnung (100) nach einem der vorangehenden Ansprüche,
wobei die Lokalspulenanordnung (100) ein erstes Teil (101) und ein zweites Teil (102) umfasst, wobei das erste Teil (101) die erste Spuleneinheit (111) und die erste Zwischeneinheit (121) umfasst, wobei das zweite Teil (102) die zweite Spuleneinheit (112) umfasst,
wobei das erste Teil (101) vom zweiten Teil (102) demontierbar ist.

5. Lokalspulenanordnung (100) nach einem der vorangehenden Ansprüche,
wobei die erste Zwischeneinheit (121) eine erste Kontakteinheit (141b) und eine zweite Kontakteinheit (144b) umfasst, wobei die erste Spuleneinheit (111) und die zweite Spuleneinheit (112) jeweils zumindest eine weitere Kontakteinheit (141a, 144a) umfassen,
wobei die erste Kontakteinheit (141b) der ersten Zwischeneinheit (121) an der zumindest einen weiteren Kontakteinheit (141a) der ersten Spuleneinheit (111) anordbar ist und die zweite Kontakteinheit (144b) der ersten Zwischeneinheit (121) an der zumindest einen weiteren Kontakteinheit (144a) der zweiten Spuleneinheit (112) anordbar ist,
wobei die erste Zwischeneinheit (121) relativ zu der ersten Spuleneinheit (111) und der zweiten Spuleneinheit (112) entlang der aneinander anordbaren Kontakteinheiten bewegbar ist.

6. Lokalspulenanordnung (100) nach Anspruch 5,
wobei die erste Zwischeneinheit (121) einen ersten Teil (131b) einer Magnetresonanzantenne (131) aufweist und die erste Spuleneinheit einen zweiten Teil (131a) der Magnetresonanzantenne (131) aufweist,
wobei der erste Teil (131b) und der zweite Teil (131a) der Magnetresonanzantenne (131) durch die erste Kontakteinheit (141b) der ersten Zwischeneinheit (121) und die zumindest eine weitere Kontakteinheit (141a) der ersten Spuleneinheit (111) verbindbar sind.

7. Lokalspulenanordnung (100) nach einem der Ansprüche 5 oder 6,
wobei die zumindest eine weitere Kontakteinheit (141a, 144a) der ersten Spuleneinheit (111) und/oder der zweiten Spuleneinheit (112) jeweils eine Oberfläche (141c, 144c) aufweist, die gegenüber einer Richtung der Relativbewegung (D) zur Zentralachse (X) der ersten Zwischeneinheit (121) um einen Neigungswinkel (β₁) geneigt ist, wobei der Neigungswinkel (β₁) größer als 0° und kleiner als 90° ist.

8. Lokalspulenanordnung (100) nach einem der Ansprüche 5 bis 7,
wobei die erste Kontakteinheit (141b) und/oder die zweite Kontakteinheit der (144b) ersten Zwischeneinheit (121) und/oder die jeweils zumindest eine weitere Kontakteinheit (141a, 144a) der ersten Spuleneinheit (111) und der zweiten Spuleneinheit (112) jeweils ein elektrisches Verbindungselement (141e, 144e) umfassen.

9. Lokalspulenanordnung (100) nach Anspruch 8,
wobei das elektrische Verbindungselement (141e, 144e) zumindest eine Landefläche (151, 152, 153) zur Aufnahme eines Gegenkontakts aufweist,
wobei der Gegenkontakt zumindest einen gefederten Kontakt und/oder Kugelkontakt und/oder Schleifkontakt umfasst.

10. Lokalspulenanordnung (100) nach einem der Ansprüche 8 oder 9,
wobei das elektrische Verbindungselement (141e, 144e) mehrere Landeflächen (151, 152, 153) zur Aufnahme eines Gegenkontakts sowie zumindest eine Korrekturschaltung (161, 162) umfasst, wobei das elektrische Verbindungselement (141e, 144e) ausgebildet ist, abhängig von einer Kontaktierung der mehreren Landeflächen (151, 152, 153) durch den Gegenkontakt die zumindest eine Korrekturschaltung (161, 162) zu aktivieren.

11. Lokalspulenanordnung (100) nach einem der vorangehenden Ansprüche,
wobei die Lokalspulenanordnung (100) eine erste Spuleneinheit (111), eine zweite Spuleneinheit (112), eine erste Zwischeneinheit (121) und eine zweite Zwischeneinheit (122) umfasst, wobei die zweite Zwischeneinheit (122) zwischen der ersten Spuleneinheit (111) und der zweiten Spuleneinheit (112) angeordnet ist,
wobei die erste Zwischeneinheit (121) und die zweite Zwischeneinheit (122) auf einander gegenüberliegenden Seiten der Lokalspulenanordnung (100) angeordnet sind.

12. Lokalspulenanordnung (100) nach einem der vorangehenden Ansprüche,
wobei die Lokalspulenanordnung (100) ein Gehäuse mit einer Außenoberfläche (S) umfasst, das derart ausgebildet, dass seine Form und Größe bei Relativbewegungen senkrecht zur Zentralachse (Z) der ersten Spuleneinheit (111), der zweiten Spuleneinheit (112) und der ersten Zwischeneinheit (121) unverändert bleibt.

13. Magnetresonanzvorrichtung (10) mit zumindest einer Lokalspulenanordnung (100) nach einem der Ansprüche 1 bis 12.

## Claims

1. A local coil arrangement (100) for examining an examination object by means of magnetic resonance, which local coil arrangement comprises
- at least a first coil unit (111) and a second coil unit (112),
- at least a rigid first intermediary unit (121),
wherein in an assembled state, the first intermediary unit is arranged between the first coil unit (111) and the second coil unit (112),
wherein the first coil unit (111), the second coil unit (112) and the first intermediary unit enclose at least partially an object receiving region (A) for accommodating the examination object,
wherein the object receiving region (A) has a central axis (Z),
wherein the first coil unit (111) and/or the second coil unit (112) and/or the first intermediary unit (121) comprise at least a portion of a magnetic resonance antenna (131-136), **characterized in that**
wherein the relative movements of the first coil unit (111) and/or the second coil unit (112) and/or the first intermediary unit (121) are positively coupled in such a manner that
the shape and/or size of the object receiving region (A) can be changed by a relative movement of the first coil unit (111) in a direction perpendicular to the central axis (Z), by a further relative movement of the second coil unit (112) in a direction perpendicular to the central axis (Z) and by a further relative movement of the first intermediary unit (121) in a direction perpendicular to the central axis (Z),
wherein the relative movements of the first coil unit (111), the second coil unit (112) and the first intermediary unit (121) are performed relative to one another.

2. The local coil arrangement (100) as claimed in one of the preceding claims,
wherein for relative movements of the first coil unit (111), of the second coil unit (112) and of the first intermediary unit (121) perpendicular to the central axis (Z), the distances between
- the central axis (Z) and the first coil unit (111),
- the central axis (Z) and the second coil unit (112), and
- the central axis (Z) and the first intermediary unit (121) change in the same sense.

3. The local coil arrangement (100) as claimed in one of the preceding claims,
wherein the first coil unit (111) and the first intermediary unit (121) have respective center planes (M₁, M₂) that intersect in the central axis (Z),
wherein the relative movements of the first coil unit (111) and of the first intermediary unit (121) perpendicular to the central axis (Z) are performed parallel to the respective center planes (M₁, M₂).

4. The local coil arrangement (100) as claimed in one of the preceding claims,
wherein the local coil arrangement (100) comprises a first part (101) and a second part (102), wherein the first part (101) comprises the first coil unit (111) and the first intermediary unit (121), wherein the second part (102) comprises the second coil unit (112),
wherein the first part (101) can be detached from the second part (102).

5. The local coil arrangement (100) as claimed in one of the preceding claims,
wherein the first intermediary unit (121) comprises a first contact unit (141b) and a second contact unit (144b),
wherein the first coil unit (111) and the second coil unit (112) each comprise at least one further contact unit (141a, 144a),
wherein the first contact unit (141b) of the first intermediary unit (121) can be arranged on the at least one further contact unit (141a) of the first coil unit (111), and the second contact unit (144b) of the first intermediary unit (121) can be arranged on the at least one further contact unit (144a) of the second coil unit (112),
wherein the first intermediary unit (121) can move relative to the first coil unit (111) and to the second coil unit (112) along the contact units, which can be arranged against one another.

6. The local coil arrangement (100) as claimed in claim 5,
wherein the first intermediary unit (121) comprises a first portion (131b) of a magnetic resonance antenna (131), and the first coil unit comprises a second portion (131a) of the magnetic resonance antenna (131),
wherein the first portion (131b) and the second portion (131a) of the magnetic resonance antenna (131) can be connected by the first contact unit (141b) of the first intermediary unit (121) and by the at least one further contact unit (141a) of the first coil unit (111).

7. The local coil arrangement (100) as claimed in either claim 5 or claim 6,
wherein the at least one further contact unit (141a, 144a) of the first coil unit (111) and/or of the second coil unit (112) each have a surface (141c, 144c) that is inclined with respect to a direction of the movement (D) relative to the central axis (X) made by the first intermediary unit (121) by an angle of inclination (β₁), wherein the angle of inclination (β₁) is greater than 0° and less than 90°.

8. The local coil arrangement (100) as claimed in one of claims 5 to 7,
wherein the first contact unit (141b) and/or the second contact unit (144b) of the first intermediary unit (121) and/or the at least one further contact unit (141a, 144a) of the first coil unit (111) and respectively of the second coil unit (112), each comprise an electrical connecting element (141e, 144e).

9. The local coil arrangement (100) as claimed in claim 8,
wherein the electrical connecting element (141, 144e) comprises at least one land (151, 152, 153) for receiving a mating contact,
wherein the mating contact comprises at least one springloaded contact and/or ball contact and/or sliding contact.

10. The local coil arrangement (100) as claimed in one of claims 8 or 9,
wherein the electrical connecting element (141e, 144e) comprises a plurality of lands (151, 152, 153) for receiving a mating contact and comprises at least one correction circuit (161, 162),
wherein the electrical connecting element (141e, 144e) is designed to activate the at least one correction circuit (161, 162) according to contact made with the plurality of lands (151, 152, 153) by the mating contact.

11. The local coil arrangement (100) as claimed in one of the preceding claims,
wherein the local coil arrangement (100) comprises a first coil unit (111), a second coil unit (112), a first intermediary unit (121) and a second intermediary unit (122), wherein the second intermediary unit (122) is arranged between the first coil unit (111) and the second coil unit (112), wherein the first intermediary unit (121) and the second intermediary unit (122) are arranged on opposite sides of the local coil arrangement (100) from each other.

12. The local coil arrangement (100) as claimed in one of the preceding claims,
wherein the local coil arrangement (100) comprises a housing having an external surface (S) that is designed such that its shape and size remain unchanged when there are relative movements of the first coil unit (111), of the second coil unit (112) and of the first intermediary unit (121) perpendicular to the central axis (Z).

13. A magnetic resonance apparatus (10) having at least one local coil arrangement (100) as claimed in one of claims 1 to 12.

## Revendications

1. Agencement (100) de bobine local pour étudier un objet à étudier au moyen de la résonance magnétique, comprenant
- au moins une premier (111) et une deuxième unité (112) de bobine,
- au moins une première unité (121) intermédiaire fixe, dans lequel, à l'état monté, la première unité intermédiaire est disposée entre la première unité (111) de bobine et la deuxième unité (112) de bobine,
dans lequel la première unité (111) de bobine, la deuxième unité (112) de bobine et la première unité intermédiaire entourent, au moins en partie, une région (A) de réception d'un objet, pour la réception de l'objet à étudier, dans lequel la région (A) de réception d'un objet a un axe (Z) central,
dans lequel la première unité (111) de bobine et/ou la deuxième unité (112) de bobine et/ou la première unité (121) intermédiaire a au moins une partie d'une antenne (131 à 136) de résonance magnétique,
**caractérisé**
**en ce que** les déplacements relatifs de la première unité (111) de bobine, de la deuxième unité (112) de bobine et de la première unité (121) intermédiaire sont couplés de manière forcée, de manière à pouvoir modifier la forme et/ou la dimension de la région (A) de réception d'un objet par un déplacement relatif de la première unité (111) de bobine dans une direction perpendiculaire à l'axe (Z) central, par un autre déplacement relatif de la deuxième unité (112) de bobine dans une direction perpendiculaire à l'axe (Z) central et par un autre déplacement relatif de la première unité (121) intermédiaire dans une direction perpendiculaire à l'axe (Z) central,
les déplacements relatifs de la première unité (111) de bobine, de la deuxième unité (112) de bobine et de la première unité (121) intermédiaire s'effectuant les uns par rapport aux autres.

2. Agencement (100) de bobine local suivant l'une des revendications précédentes,
dans lequel, lors du déplacement relatif perpendiculaire à l'axe (Z) central de la première unité (111) de bobine, de la deuxième unité (112) de bobine et de la première unité (121) intermédiaire, les distances entre
- l'axe (Z) central et la première unité (111) de bobine,
- l'axe (Z) central et la deuxième unité (112) de bobine ainsi que
- l'axe (Z) central et la première unité (121) intermédiaire se modifient dans le même sens.

3. Agencement (100) de bobine local suivant l'une des revendications précédentes,
dans lequel la première unité (111) de bobine et la première unité (121) intermédiaire ont chacune un plan (M₁, M₂) médian, qui se coupent dans l'axe (Z) central,
dans lequel les déplacements relatifs, perpendiculaires à l'axe (Z) central, de la première unité (111) de bobine et de la première unité (121) intermédiaire s'effectuent parallèlement aux plans (M₁, M₂) médians respectifs.

4. Agencement (100) de bobine local suivant l'une des revendications précédentes,
dans lequel l'agencement (100) de bobine local comprend une première partie (101) et une deuxième partie (102), la première partie (101) comprenant la première unité (111) de bobine et la première unité (121) intermédiaire, la deuxième partie (102) comprenant la deuxième unité (112) de bobine,
dans lequel la première partie (101) peut être démontée de la deuxième partie (102).

5. Agencement (100) de bobine local suivant l'une des revendications précédentes,
dans lequel la première unité (121) intermédiaire comprend une première unité (141b) de contact et une deuxième unité (144b) de contact, la première unité (111) de bobine et la deuxième unité (112) de bobine comprenant chacune au moins une autre unité (141a, 144a) de contact,
dans lequel la première unité (141b) de contact de la première unité (121) intermédiaire peut être disposée sur la au moins un autre unité (141) de contact de la première unité (111) de bobine et la deuxième unité (144b) de contact de la première unité (121) intermédiaire peut être disposée sur la au moins une autre unité (144a) de contact de la deuxième unité (112) de bobine,
dans lequel la troisième unité (121) intermédiaire est mobile par rapport à la première unité (111) de bobine et la deuxième unité (112) de bobine est mobile le long des unités de contact pouvant être mises l'une contre l'autre.

6. Agencement (100) de bobine local suivant la revendication 5,
dans lequel la première unité (121) intermédiaire a une première partie (131b) d'une antenne (131) de résonance magnétique et la première unité de bobine a une deuxième partie (131a) de l'antenne (131) de résonance magnétique, dans lequel la première partie (131b) et la deuxième partie (131a) de l'antenne (131) de résonance magnétique peuvent être reliées par la première unité (141b) de contact de la première unité (121) intermédiaire et par la au moins une autre unité (141a) de contact de la première unité (111) de bobine.

7. Agencement (100) de bobine local suivant l'une des revendications 5 ou 6,
dans lequel la au moins une autre unité (141a, 144a) de contact de la première unité (111) de bobine et/ou de la deuxième unité (112) de bobine ont chacune une surface (141c, 144c), qui est, par rapport à une direction du déplacement (D) relatif, par rapport à l'axe (X) central de la première unité (121) intermédiaire, inclinée d'un angle (β₁) d'inclinaison, l'angle (β₁) d'inclinaison étant plus grand que 0° et plus petit que 90°.

8. Agencement (100) de bobine local suivant l'une des revendications 5 à 7,
dans lequel la première unité (141b) de contact et/ou la deuxième unité (144b) de contact, la première unité (121) intermédiaire et/ou la, respectivement, au moins une autre unité (141a, 144a) de contact de la première unité (111) de bobine et de la deuxième unité (112) de bobine comprennent chacune un élément (141e, 144e) de liaison électrique.

9. Agencement (100) de bobine local suivant la revendication 8,
dans lequel l'élément (141e, 144e) de liaison électrique a au moins une surface (151, 152, 153) de piste de réception d'un contact antagoniste,
dans lequel le contact antagoniste comprend au moins un contact à ressort et/ou un contact à bille et/ou un contact à balai.

10. Agencement (100) de bobine local suivant l'une des revendications 8 ou 9,
dans lequel le premier élément (141e, 144e) de liaison électrique comprend plusieurs surfaces (151, 152, 153) de piste pour recevoir un contact antagoniste, ainsi qu'au moins un circuit (161, 162) de correction, l'élément (141e, 144e) de liaison électrique étant constitué pour, en fonction d'une mise en contact des plusieurs surfaces (151, 152, 153) de piste, activer par le contact antagoniste, le au moins un circuit (161, 162) de correction.

11. Agencement (100) de bobine local suivant l'une des revendications précédentes,
dans lequel l'agencement (100) de bobine local comprend une première unité (111) de bobine, une deuxième unité (112) de bobine, une première unité (121) intermédiaire et une deuxième unité (122) intermédiaire, la deuxième unité (122) intermédiaire étant disposée entre la première unité (111) de bobine et la deuxième unité (112) de bobine,
dans lequel la première unité (121) intermédiaire et la deuxième unité (122) intermédiaire sont disposées sur des côtés opposés l'un à l'autre de l'agencement (100) de bobine local.

12. Agencement (100) de bobine local suivant l'une des revendications précédentes,
dans lequel l'agencement (100) de bobine local comprend un boîtier ayant une surface (S) extérieure, constituée de manière à ce que sa forme et sa dimension restent inchangées s'il se produit des déplacements relatifs perpendiculairement à l'axe (Z) central de la première unité (111) de bobine, de la deuxième unité (112) de bobine et de la première unité (121) intermédiaire.

13. Système (10) à résonance magnétique, comprenant au moins un agencement (100) de bobine local suivant l'une des revendications 1 à 12.
